# EUROPEAN PATENT SPECIFICATION

(11) **EP 2 769 744 B1**
(45) Date of publication and mention of the grant of the patent: **08.02.2017**
(21) Application number: 14155719.9
(22) Date of filing: 19.02.2014
(51) Int. Cl.: A61M 1/00

(54) **Medical product and medical set for the drainage of pathological accumulation of fluids**
Medizinisches Produkt und medizinisches Set zur Drainage von pathologischen Ansammlungen von Fluiden
Produit médical et ensemble médical pour le drainage de l'accumulation pathologique de fluides

(30) Priority: 21.02.2013 DE 102013202849
(43) Date of publication of application: 27.08.2014
(73) Proprietor: AESCULAP AG, 78532 Tuttlingen (DE)
(72) Inventor: Abele, Wolfgang, 78532 Tuttlingen (DE); Odermatt, Erich, 8200 Schaffhausen (CH)
(74) Representative: Patentanwälte Ruff, Wilhelm, Beier, Dauster & Partner mbB

(56) References cited:
- CA-A1- 2 829 512
- US-A1- 2001 029 956

## Description

### Scope of Application and Prior Art

The invention concerns a medical product and a medical set respectively for the drainage of pathological accumulation of fluids such as accumulations of ichor.

So-called endoluminal vacuum therapy is a therapeutic option for infections of body cavities. In this type of therapy, a special wound drainage system is used that essentially comprises an open pore sponge for absorbing ichors and a drainage tube for removing or draining the absorbed ichors. This type of wound drainage system is marketed by the Applicant under the name Endo-Sponge^{®}.

As a rule, the sponge is placed by means of an overtube in a body cavity to be treated. If the overtube is too short or has too large a diameter, the sponge must be pushed or pulled into the cavity. This usually requires the use of a grasping instrument such as grasping forceps of an endoscope. However, as the drainage tube cannot be pulled using such a grasping instrument because of its thickness and smooth surface, the grasping instrument is usually positioned on the sponge for placement in the body cavity. However, this causes a risk that the sponge may be torn out.

In addition, stenosis of hollow organs such as the rectum or oesophagus may make placement of the sponge by means of an overtube difficult or even impossible.

Document CA 2829512 discloses a sponge drainage device.

The invention of WO 2009/140376 A1 uses a filament instead of a sponge. The filament is placed in a subcutaneous wound cavity in the form of a mass using a delivery instrument. Placement is carried out manually and requires a certain degree of skill and dexterity on the part of the treating physician.

US Patent No. 2007/0219497 A1 discloses a drainage system suitable for endoluminal vacuum therapy that is placed by means of a guide wire.

### Problem and Solution

Against this backdrop, the problem to be solved by the present invention is to provide a medical product that avoids the drawbacks known in prior art, and more particularly allows damage-free placement of the product in the body of a human or animal patient, particularly in a body cavity of the patient.

The invention solves this problem by comprising a medical product having the characteristics of independent claim 1 and a medical set having the characteristics of independent claim 17. Preferred embodiments of the product are defined in dependent claims 2 to 16. The wording of all these claims is incorporated herein by specific reference.

The product according to the invention is a medical product for the drainage of pathological accumulation of fluids such as accumulations of ichor.

The product comprises a fluid collection element and a fluid communication element, with the fluid communication element being at least partially and preferably only partially arranged inside the fluid collection element.

The fluid communication element preferably extends along the entire length of the fluid collection element.

Advantageously, the fluid communication element is connected to the fluid collection element in such a manner as to conduct fluid.

More particularly, the medical product is characterized in that a pulling element for the placement of the medical product, particularly the fluid collection element, in a human or animal body, and preferably a human or animal body cavity, is arranged inside the fluid communication element.

The invention is characterized by the following advantages:
- The pulling element allows damage-free placement of the medical product, and more particularly the fluid collection element.
- Arrangement of the pulling element in the fluid communication element prevents interference with any required adjustment of the fluid collection element, particularly reduction in the size thereof.
- After adjustment of the fluid collection element, and more particularly reduction in size thereof, the distance between the fluid collection element and the pulling element may be easily locked in by the treating physician.
- In the case of a pulling element connected in a detachable manner to the fluid communication element, the pulling element may be withdrawn from, and more particularly, pulled out of the fluid communication element after the fluid collection element is put in place, so that there is no risk that the pulling element will cause irritation, form adhesions with the surrounding tissue, and/or lead to tissue tearing resulting from said adhesions when said pulling element is removed.

For the purposes of the present invention, the term "fluid collection element" is to be understood as referring to an element that is capable of taking up, and more preferably, suctioning up and collecting pathologically accumulated fluids, as well as discharging said taken up and collected fluids, as a rule via application of a negative pressure, suction, or vacuum, into the fluid communication element.

For the purposes of the present invention, the term "fluid communication element" is to be understood as referring to an element that allows fluid communication to be established between the fluid collection element and a fluid-collecting component such as a collecting vessel.

For the purposes of the present invention, the term "pathological accumulation of fluids" is to be understood as referring to the pathological accumulation of body fluids and/or gases. Examples of body fluids include ichors, exudates, purulent fluids, and intestinal contents.

For the purposes of the present invention, the term "body cavity" is preferably to be understood as referring to a so-called wound cavity. This includes naturally acquired or pathological bulges that may be filled primarily with ichors. In such cases, the pathological bulges are often the result of surgical procedures, particularly of anastomoses such as rectal anastomoses. In such cases, bulges may form in the ligature or suture area and rapidly increase in size because of infections. The term "insufficiency cavity" is frequently used in the case of bulges in the suture area. However, pathological bulges may also be caused by infections and/or inflammations resulting in the formation of fistulae, for example.

For the purposes of the present invention, the term "pulling element" is to be understood as referring to an element that allows placement of the medical product, particularly the fluid collection element, in a human or animal body or a corresponding body cavity by pulling and/or subsequent application of tensile force.

For the purposes of the present invention, the term "proximal end" refers to an end facing toward the trunk of the treating physician, while the term "distal end" refers to an end facing away from the trunk of the treating physician.

In a preferred embodiment, the pulling element is arranged in a cavity, preferably a conduit, of the fluid communication element, or in a cut in the fluid communication element. The cavity may be configured in cylindrical or essentially cylindrical, and preferably in circular cylindrical or essentially circular cylindrical form. Moreover, the cavity is preferably arranged parallel to a longitudinal axis of the fluid communication element. Generally, the cavity can extend continuously, i.e. without interruptions, in longitudinal direction of the fluid communication element.

The pulling element is preferably in contact with and preferably connected to the fluid communication element.

It is particularly advisable to connect the pulling element to the fluid communication element in a detachable manner. This makes it possible to remove the pulling element from the fluid communication element, and more particularly to pull it out of said element, after placement of the medical product, and to avoid the risk of irritation, adhesion, or tearing out of tissue after the pulling element has been left in place *in vivo.*

In a further embodiment, the pulling element is connected to an end, and preferably a proximal end, of the fluid communication element.

Connection of the pulling element to the fluid communication element, and preferably to a proximal end of the fluid communication element, can generally be carried out by means of form closure, force closure, and/or material bond.

More particularly, connection of the pulling element to the fluid communication element, preferably to a proximal end of the fluid communication element, may be achieved by means of a fixation device such as an adhesive, a sleeve, a plug, a knot, a filament or the like.

In a preferred embodiment, the pulling element has a fixation device in the form of (a) knot(s) at its proximal end, and the fluid communication element has a slit at its proximal end. This makes it possible for the pulling element to move in the slit up to the at least one knot(s), thus allowing the knot(s) to keep the pulling element from slipping and preventing premature release of the pulling element from the fluid communication element. Particularly, the fixation device may be in the form of several knots, allowing the length of the pulling element to be adjusted in a targeted manner and adapted to conform to patient-specific factors.

In a further embodiment, the pulling element protrudes from a distal and/or proximal end of the fluid communication element. Protrusion of the pulling element from the distal end of the fluid communication element is advantageous mainly because this makes it easier to grasp the pulling element, and thus the medical product, under visual control by means of a grasping device such as forceps (of an endoscope). This in turn allows easier and safer placement of the medical product.

In a particularly preferred embodiment, the pulling element is formed as a loop. The loop configuration is advantageous because it makes it far easier to grasp the pulling element and thus the medical product.

In a particularly preferred embodiment, the pulling element protrudes from a distal end of the fluid communication element in the form of a loop.

The loop may be fixed in place by at least one fixation device. A suitable fixation device may be selected from the group comprising knots, adhesives, sleeves, plugs, filaments, and combinations thereof.

In a further embodiment, a fixed end of the loop protrudes from a proximal end of the fluid communication element.

In principle, the pulling element may be configured in textile or non-textile form. However, a textile configuration of the pulling element is preferred. In other words, a textile structure of the pulling element is preferred.

Preferably, the pulling element comprises at least one filament or is preferably configured in the form of at least one filament (so-called pulling filament).

Particularly, the pulling element may be selected from the group comprising a monofilament, pseudomonofilament, or a multifilament, with a braided multifilament being preferred.

In a further embodiment, the pulling element is composed of polymer filaments. With respect to suitable filament materials, all of the polymers specified below are incorporated herein by reference.

Particularly, the pulling element may include or be composed of a polymer selected from the group comprising polyolefins, polyester, polyamides, polyurethanes, polyhydroxyalkanoates, copolymers thereof, salts thereof, stereoisomers thereof, and mixtures, preferably blends, thereof.

For example, the pulling element may contain or be composed of a polymer selected from the group comprising polyethylene, polypropylene, polyethylene terephthalate, polypropylene terephthalate, polybutylene terephthalate, Nylon 6, Nylon 6-6, Nylon 6-12, polyglycolide, polylactide, poly-ε-caprolactone, polytrimethylene carbonate, poly-3-hydroxybutyrate, poly-4-hydroxybutyrate, copolymers thereof, salts thereof, stereoisomers thereof and mixtures, preferably blends, thereof.

In a further embodiment, the pulling element is a metallic pulling element, preferably a metal filament (wire) or a metal spiral. The pulling element may be composed of a metal selected from the group comprising gold, iridium, platinum, tantalum, nickel, titanium, and combinations or alloys thereof. Nickel-titanium-alloys, particularly nitinol, are preferred.

In a further embodiment, the pulling element may be configured in the form of a looped filament, wherein the filament loop preferably protrudes from the fluid communication element. In this case, the filaments may fully extend through the fluid communication element two times. The two filament sections running in opposite directions may be connected to each other, particularly by means of an adhesive, a sleeve, a clip, or a further, preferably thin filament. The loop preferably protrudes from a distal end of the fluid communication element, and the fixed end of the loop preferably protrudes from a proximal end of said element, preferably in the form of two fixed filament ends. With respect to suitable filament materials, the polymers, metals, and metal alloys and/or combinations thereof specified above are fully incorporated herein by reference.

In a further embodiment, the pulling element is stiffened. For example, the pulling element may be stiffened by means of a coating.

Generally, the pulling element may not be radiopaque.

In a preferred embodiment, however, the pulling element is radiopaque. A radiopaque pulling element is advantageous because this makes it possible to place the medical product using suitable imaging techniques. For example, the pulling element may be provided for this purpose with radiopaque particles such as barium sulphate particles. Alternatively, the pulling element may comprise a radiopaque metal or a radiopaque metal alloy or may be composed of such a metal or metal alloy. The radiopaque metal and/or radiopaque metal alloy is preferably selected from the group comprising gold, iridium, platinum, tantalum, nickel, titanium, and alloys thereof, preferably nitinol.

According to the invention, moreover, in order to achieve sufficient and/or optimum sliding friction, depending particularly on the material used and the length, thickness, and/or texture of the pulling element, said element may be coated with a friction-reducing material. In alternative embodiments, the inner surface of a cavity, preferably a conduit, or the inner surfaces of a cut of the fluid communication element may be coated with a friction-reducing material.

In principle, the size and shape of the fluid collection element are not significant, provided that they can be adjusted to conform to a body area to be treated, and preferably to a body cavity to be treated (for example, a rectal fistula resulting from insufficiency of a rectal anastomosis). Although it may be advantageous to use a fluid collection element that is adapted from the beginning to the size and shape of the body area to be treated, it may also be advantageous, particularly in the case of body areas that are not easily accessible, to adapt the fluid collection element only immediately prior to placement of the medical product.

In order to improve handling properties, the fluid collection element may be provided with a flocking material. The flocking material is typically attached to the fluid collection element and/or its surface by means of an adhesive layer that covers the surface of the fluid collection element at least partially, and preferably only partially. For example, a suitable adhesive may be selected from the group comprising melt adhesives, preferably heat-activatable adhesives and/or hotmelt adhesives, polyvinyl alcohol, polyvinylacetate, polyvinylchloride, polyvinylpropionate, polyacrylate, cyanoacrylate, modified vinylacetate copolymer polyester-elastomer, polyurethane-elastomer, vinylpyrrolidone-vinylacetate copolymer, polycarbonate, rubber, acrylonitrile-butadiene-styrene copolymer, hydrogel based on polyethylene glycol, resin, wax, preferably beeswax, and mixtures thereof.

The flocking material is preferably configured as flock fibres.

The flocking material may be selected from the group comprising polyolefins, polyamides, polyimides, polyesters, polyurethanes, preferably thermoplastic polyurethanes, polyhydroxyalkanoates, proteins, polysaccharides, copolymers thereof, salts thereof, stereoisomers thereof and mixtures, preferably blends, thereof.

Particularly, the flocking material may be selected from the group comprising polyethylene, low-density polyethylene, high-density polyethylene, high-molecular-weight polyethylene, ultra-high-molecular-weight polyethylene, polypropylene, polyethylene terephthalate, polypropylene terephthalate, polybutylene terephthalate, Nylon 6, Nylon 6-6, Nylon 6-12, Nylon 12, silk, polytetrafluoroethylene, polyvinylidene difluoride, polytetrafluoropropylene, polyhexafluoropropylene, polyvinyl alcohol, polyglycolide, polylactide, polydioxanone, poly-3-hydroxybutyrate, poly-4-hydroxybutyrate, polytrimethylene carbonate, poly-ε-caprolactone, collagen, gelatine, elastin, reticulin, fibronectin, laminin, fibrin, fibrinogen, albumin, starch, amylose, amylopectin, dextran, viscose, cotton, cellulose, methylcellulose, carboxymethylcellulose, chitosan, hyaluronic acid, dextran sulphate, heparin, heparan sulphate, chondroitin sulphate, dermatan sulphate, copolymers thereof, salts thereof, stereoisomers thereof and mixtures, preferably blends, thereof.

The fluid collection element has a cylindrical form in preferred embodiments. For example, the fluid collection element may be configured in the form of a cylinder having a circular or non-circular cross-section, particularly an oval, triangular, square, trapezoid, rhomboid, pentagonal, or hexagonal cross-section. A circular cylindrical or essentially circular cylindrical configuration of the fluid collection element is preferred.

In a preferred embodiment, the fluid communication element is arranged in a cavity, preferably a conduit, of the fluid collection element, or in a cut in the fluid collection element. The cavity may be configured in cylindrical or essentially cylindrical, and preferably in circular cylindrical or essentially circular cylindrical form. Moreover, the cavity is preferably arranged parallel to a longitudinal axis, and particularly along an axis of symmetry, of the fluid collection element. Generally, the cavity can extend continuously, i.e. without interruptions, along the fluid collection element.

The fluid collection element and the fluid communication element may also be configured such that they can be positioned (moved) with respect to each other.

In a further embodiment, the fluid communication element is configured in the fluid collection element in such a manner that the distal end of the fluid collection element and the distal end of the fluid communication element are aligned (flush) with each other.

In principle, however, the fluid communication element may also be arranged in the fluid collection element in such a manner that the distal end of the fluid collection element and the distal end of the fluid communication element are not aligned (not flush) with each other.

Alternatively, the fluid collection element and the fluid communication element may be connected to each other. The connection can be based on form closure, force closure, and/or material bond.

The fluid collection element may also be moulded onto the fluid communication element. For example, the fluid collection element may be glued, sewn, welded, or foamed onto the fluid communication element.

Generally, at least one part, and preferably only one part, of the fluid communication element is surrounded by or encased in the fluid collection element.

The fluid collection element and the fluid communication element may also be connected by means of a seam. Preferably, the seam does not pass through a cavity of the fluid communication element so as to prevent to the extent possible any interference with arrangement of the pulling element in the fluid communication element.

In a preferred embodiment, the fluid collection element is configured in open porous or open pore form.

The fluid collection element may comprise pores having a diameter of 100 µm to 1500 µm, preferably 200 µm to 1000 µm, and more preferably 400 µm to 800 µm.

Moreover, the fluid collection element may contain additives, particularly selected from the group comprising antimicrobial, and preferably antibiotic agents, antiseptic agents, disinfecting agents, anti-inflammatory agents, odour-inhibiting agents and mixtures thereof.

In preferred embodiments, the fluid collection element is composed of a spacer material.

In principle, the fluid collection element may be resorbable, partially resorbable, or non-resorbable.

For example, the fluid collection element may comprise and/or may be composed of a non-resorbable polymer, wherein the polymer is preferably selected from the group comprising polyolefins such as polypropylene, polyethylene, polyethylene terephthalate, polypropylene terephthalate, polybutylene terephthalate, polyurethane, polyvinyl alcohol, polyester, copolymers thereof and mixtures, preferably blends, thereof. Because of its biocompatibility, polyurethane is particularly preferred as a material for the fluid collection element.

The fluid collection element preferably comprises and/or is preferably composed of polyurethane.

In a further embodiment, the polyurethane may be an aliphatic polyurethane.

The polyurethane may also be a linear, branched, or cyclic polyurethane.

According to the invention, a linear aliphatic polyurethane is preferred.

The polyurethane may also be composed of macromolecular and/or low-molecular-weight aliphatic diols and aliphatic diisocyanates. Examples of suitable macromolecular diols are polycarbonates such as 1,6-hexanediol polycarbonate. Suitable low-molecular-weight diols may be selected from the group comprising 2,2,4-trimethylhexanediol, 2,4,4-trimethylhexanediol, 1,4-butanediol, and mixtures thereof. Suitable preferred aliphatic diisocyanates are cycloaliphatic diisocyanates such as 4,4-dicyclohexylmethane-4,4-diisocyanate or 1,4-cyclohexyldiisocyanate. The polyurethane may also be produced from various diols and/or diisocyanates.

In a further embodiment, the polyurethane is selected from the group comprising aliphatic polycarbonate urethane, silicone polycarbonate urethane, polyether urethane, silicone polyether urethane, polyurethane ether, and mixtures thereof.

In an alternative embodiment, the fluid collection element comprises and/or is composed of a resorbable polymer, wherein the polymer is preferably selected from the group comprising polyglycolide, polylactide, poly-ε-caprolactone, trimethylene carbonate, poly(para-dioxanone), poly-3-hydroxybutyrate, poly-4-hydroxybutyrate, copolymers thereof, salts thereof, stereoisomers thereof and mixtures, preferably blends, thereof.

In a particularly preferred embodiment, the fluid collection element is configured in the form of a sponge or foam, particularly an open porous sponge or an open porous foam.

In a further embodiment, the fluid communication element is composed of an intrinsically fluid-tight, and preferably a liquid-tight material. For example, the fluid communication element may be composed of polyethylene, polypropylene, polyvinylchloride, polyurethane, or mixtures, and preferably blends, thereof.

The fluid communication element is preferably configured in the form of at least one tube, and preferably at least one drainage tube.

Generally, the fluid communication element can be configured in the form of a tube, and preferably a drainage tube.

According to the invention, the fluid communication element may also comprise several tubes. For example, the fluid communication element may comprise two tubes, preferably with one tube being provided for draining or removal of the accumulated fluids and the other being provided for rinsing of the fluid collection element. With respect to fluids suitable for this purpose, the rinsing fluids specified below are also incorporated herein by reference.

Alternatively, the fluid communication element may be configured with multiple lumens and/or conduits. For this purpose, an original cavity (and/or original lumen), and preferably an original conduit, of the fluid communication element may be divided by means of one or if necessary several separating walls into two or more cavities, and preferably conduits.

According to the invention, the fluid communication element may be provided both for drainage and for rinsing purposes. For rinsing purposes, the fluid communication element may be charged with a rinsing fluid, selected for example from the group comprising saline solution, buffer solution, antimicrobially active solution, anti-inflammatory solution, odour-inhibiting solution, and mixtures thereof.

In order to ensure particularly that a fluid-conducting connection is established between the fluid collection element and the fluid communication element, in a further embodiment, the fluid communication element is provided with openings, particularly holes, pores, perforations, etc., and/or cuts, particularly helical cuts.

In a preferred embodiment, the openings and/or cuts are located in an area of the fluid communication element arranged inside the fluid collection element, preferably at the distal end of the fluid communication element. This makes it possible to apply uniform negative pressure to the fluid collection element in a particularly advantageous manner. The openings and/or cuts also allow more rapid and thus more efficient drainage of the fluids taken up by the fluid collection element. In this case, the openings and/or cuts may be provided only in the area of the distal end of the fluid communication element.

In a further embodiment, the medical product consists of the fluid collection element, the fluid communication element and the pulling element, and optionally of additives. With regard to further features and advantages, reference is entirely made to the present description.

In a further embodiment, the fluid communication element is connected at its proximal end to a suction or vacuum source, particularly a suction or vacuum pump. The suction or vacuum source typically produces negative pressure or suction of 25 mmHg to 600 mmHg, preferably 25 mmHg to 200 mmHg, more preferably 50 mmHg to 150 mmHg, and even more preferably 80 mmHg to 125 mmHg. This makes it possible to achieve particularly rapid cleansing, particularly of infected body cavities, and subsequent rapid formation of granulation tissue. The therapeutic result can be influenced in a particularly advantageous manner by finely adjusting the negative pressure or suction.

It is particularly advantageous if the suction or vacuum source is a portable suction or vacuum source. This makes it possible to maintain patient mobility during treatment.

Generally, the product according to the invention, particularly the fluid collection element, may be provided for indwelling use over a period of several hours to several days in a human or animal body or a corresponding body cavity thereof. The product, particularly the fluid collection element, is typically replaced every 48 to 96 hours.

The amounts of fluid discharged are normally collected in collection vessels provided for this purpose, such as canisters or vacuum flasks. Generally, a suction or vacuum source, preferably of the type described above, can be connected in series prior to the collection vessels and is preferably connected to said vessels in a suitable manner via appropriate connecting tubes. In order to prevent contamination of the suction or vacuum source, a sterile filter may be provided between the collection vessels and the suction or vacuum source.

In a further embodiment, the medical product is provided with a grasping device for the pulling element, which preferably protrudes in the form of a loop from a distal end of the fluid communication element.

The grasping device may be connected to the pulling element.

For example, the grasping device may be fixed in the loop of a loop-shaped pulling element or may be freely movable in the loop of such a pulling element.

In principle, the grasping device may be of any conceivable geometric shape. However, said device preferably has a cylindrical or ball-shaped configuration.

In a particularly preferred embodiment, the medical product is configured as a drainage device or drainage system.

The medical product is preferably provided for conducting vacuum therapy, particularly intracavitary and/or intraluminal vacuum therapy.

In a preferred embodiment, the medical product, particularly the fluid collection element, the fluid communication element, and/or the pulling element, is in sterile and specially packaged (tailored) form.

The present invention also concerns a medical set or kit for the drainage of pathological accumulation of fluids such as accumulations of ichor, comprising a spatially separate fluid collection element, fluid communication element, and pulling element.

The medical set or kit may also comprise a grasping device for the pulling element.

Moreover, the medical set or kit may comprise a grasping device such as grasping forceps for the pulling element.

In order to avoid unnecessary repetition, with regard to further characteristics and advantages of the set or kit components described above, particularly those of the fluid collection element, fluid communication element, and pulling element, reference is entirely made to the present description.

Further characteristics and advantages of the invention are given in the following description of preferred embodiments in the form of figures, figure descriptions, and the subclaims. These may concern individual embodiments of characteristics or embodiments combining several characteristics. The embodiments described serve only to explain the present invention, which is by no means limited to said embodiments.

### Description of the Figures

The figures are schematic diagrams of the following:
Fig. 1: a (ready-to-use) embodiment of the product according to the invention and
Fig. 2: an enlarged view of the components of an embodiment of the product according to the invention:

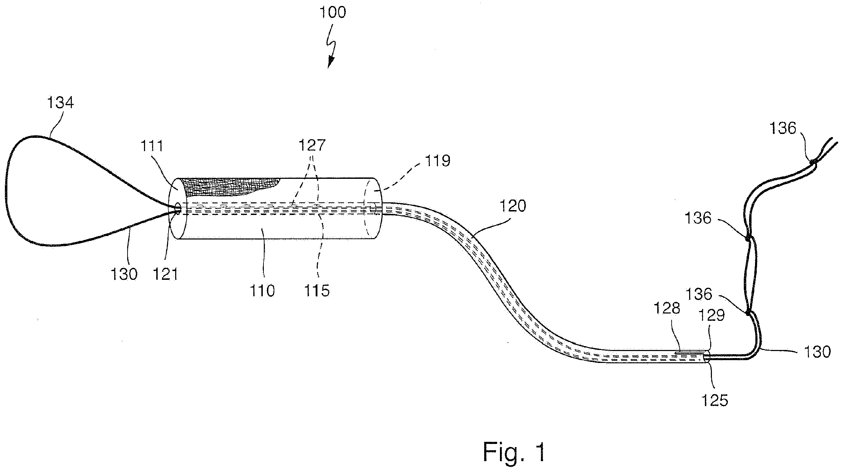

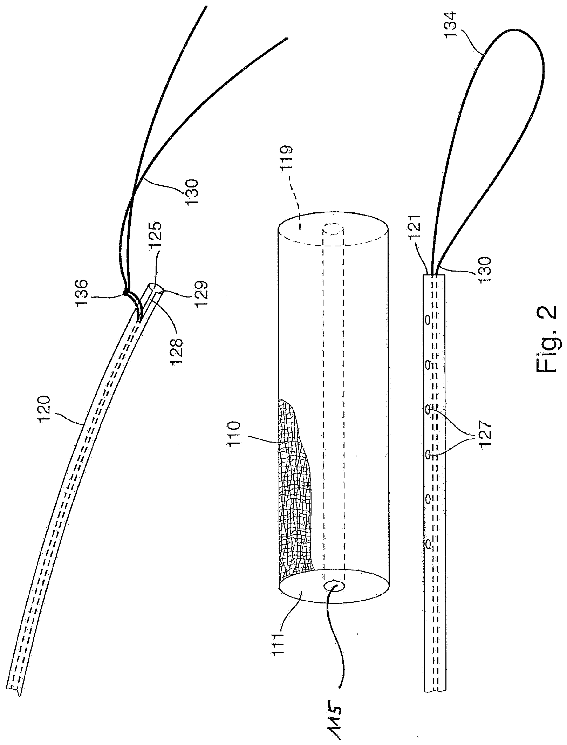

Figs. 1 and 2 show a medical product 100 according to the present invention. The product 100 is used for the drainage of pathological accumulation of fluids such as accumulations of ichor from the human or animal body, and preferably human or animal body cavities.

The product 100 has a fluid collection element 110, a fluid communication element 120, and a pulling element 130.

The fluid collection element 110 having a distal end 111 and a proximal end 119 is preferably composed of open pore foam, particularly open porous polyurethane foam. The fluid collection element 110 has a conduit-shaped cavity 115 extending continuously in a longitudinal direction that contains the fluid communication element 120, preferably in a sectional manner.

The fluid communication element 120 also has a distal end 121 and a proximal end 129.

In order to allow communication of fluids, particularly ichors, between the fluid collection element 110 and the fluid communication element 120, the latter element is provided with openings 127 in the area of its distal end 121. The fluids taken up by the fluid collection element 110, particularly when negative pressure or a vacuum is applied, may be discharged via the openings 127.

The fluid communication element 120 also has a conduit-shaped cavity 125 that extends continuously in its longitudinal direction.

The pulling element 130, which is preferably configured in the form of a looped filament, is positioned in the cavity 125. The pulling element 130 is preferably longer than the fluid communication element 120 so that the pulling element 130 extends beyond the ends 121; 129 of the fluid communication element 120.

The pulling element 130 is preferably configured in filament form or composed of filaments. In other words, the pulling element 130 is preferably a pulling filament.

At the distal end 121, the pulling element 130 preferably protrudes in the form of a loop 134. A loop-shaped configuration of the pulling element 130 at the distal end 121 is particularly advantageous in facilitating grasping of the pulling element 130 and thus placement of the product 100 as a whole.

The pulling element 130 is preferably fastened to the proximal end 129 of the fluid communication element 120. In this case, the pulling element 130 may have one or - as shown in Figs. 1 and 2 - several fixation devices 136.

If the fixation devices 136 are in the form of knots, it has been found to be advantageous for the fluid communication element 120 to have a slit 128 at its proximal end 129. In the slit 128, the pulling element 130 can move up to the respective knots, and said knots keep the pulling element 130 from slipping, thus preventing undesirable (premature) detachment of the connection between the pulling element 130 and the fluid communication element 120.

In the intended application, the fluid communication element 120 is inserted into the cavity 115 of the fluid collection element 110. In this case, the pulling element 130 may already be positioned in the cavity 125 of the fluid communication element 120. Alternatively, however, the pulling element 130 may also be placed in the fluid communication element 120 after said fluid communication element 120 has been inserted into the fluid collection element 110.

The product 100 according to the invention is chiefly suitable for use in vacuum therapy, particularly endoluminal and/or intracavitary vacuum therapy.

## Claims

1. Medical product for the drainage of pathological accumulation of fluids such as accumulations of ichor, comprising a fluid collection element (110) and a fluid communication element, wherein the fluid communication element (120) is at least partially arranged inside the fluid collection element and is connected therewith so as to conduct fluid, wherein a pulling element (130) for placing the product in a human or animal body is arranged inside the fluid communication element, **characterized in that** the pulling element protrudes from a distal end of the fluid communication element in the form of a loop.

2. Medical product according to claim 1, **characterized in that** the fluid communication element extends along the entire length of the fluid collection element.

3. Medical product according to claim 1 or 2, **characterized in that** the pulling element is arranged in a cavity or cut of the fluid communication element, wherein the cavity or cut extends continuously along the fluid communication element.

4. Medical product according to one of the preceding claims, **characterized in that** the pulling element is connected to the fluid communication element, preferably to a proximal end thereof.

5. Medical product according to one of the preceding claims, **characterized in that** a fixed end of the loop protrudes from a proximal end of the fluid communication element.

6. Medical product according to one of the preceding claims, **characterized in that** the pulling element is arranged as textile, in particularly at least one filament.

7. Medical product according to one of the preceding claims, **characterized in that** the pulling element is arranged as a looped filament, wherein said looped filament is longer than the fluid communication element.

8. Medical product according to one of the preceding claims, **characterized in that** the fluid communication element is arranged in a cavity of the fluid collection element, wherein the cavity extends continuously along the length of the fluid collection element.

9. Medical product according to one of the preceding claims, **characterized in that** the fluid communication element is arranged in the fluid collection element in such a manner that the distal end of the fluid collection element and the distal end of the fluid communication element are aligned (flush) with each other.

10. Medical product according to one of the preceding claims, **characterized in that** the fluid collection element is configured as an open pored sponge.

11. Medical product according to one of the preceding claims, **characterized in that** the fluid collection element is composed of polyurethane, particularly linear and preferably aliphatic polyurethane.

12. Medical product according to one of the preceding claims, **characterized in that** the fluid communication element is configured in the form of a drainage tube.

## Patentansprüche

1. Medizinisches Produkt zur Ableitung von pathologischen Fluidansammlungen wie Wundsekretansammlungen, umfassend einen Fluidsammelkörper (110) und einen Fluidkommunikationskörper, wobei der Fluidkommunikationskörper (120) wenigstens teilweise in dem Fluidsammelkörper angeordnet ist und fluidleitend mit diesem verbunden ist, wobei in dem Fluidkommunikationskörper ein Zugelement (130) zur Platzierung des Produktes in einen menschlichen oder tierischen Körper angeordnet ist, **dadurch gekennzeichnet, dass** das Zugelement aus einem distalen Ende des Fluidkommunikationskörpers in Form einer Schlaufe heraustritt.

2. Medizinisches Produkt nach Anspruch 1, **dadurch gekennzeichnet, dass** sich der Fluidkommunikationskörper entlang der vollständigen Länge des Fluidsammelkörpers erstreckt.

3. Medizinisches Produkt nach Anspruch 1 oder 2, **dadurch gekennzeichnet, dass** das Zugelement in einem Hohlraum oder Einschnitt des Fluidkommunikationskörpers angeordnet ist, wobei sich der Hohlraum bzw. Einschnitt durchgehend in Längsrichtung des Fluidkommunikationskörpers erstreckt.

4. Medizinisches Produkt nach einem der vorhergehenden Ansprüche, **dadurch gekennzeichnet, dass** das Zugelement mit dem Fluidkommunikationskörper, vorzugsweise mit einem proximalen Ende davon, verbunden ist.

5. Medizinisches Produkt nach einem der vorhergehenden Ansprüche, **dadurch gekennzeichnet, dass** ein fixiertes Ende der Schlaufe aus einem proximalen Ende des Fluidkommunikationskörpers heraustritt.

6. Medizinisches Produkt nach einem der vorhergehenden Ansprüche, **dadurch gekennzeichnet, dass** das Zugelement textil, insbesondere in Form wenigstens eines Fadens, ausgestaltet ist.

7. Medizinisches Produkt nach einem der vorhergehenden Ansprüche, **dadurch gekennzeichnet, dass** das Zugelement als geschlaufter Faden ausgebildet ist, wobei die Fadenschlaufe länger ist als der Fluidkommunikationskörper.

8. Medizinisches Produkt nach einem der vorhergehenden Ansprüche, **dadurch gekennzeichnet, dass** der Fluidkommunikationskörper in einem Hohlraum des Fluidsammelkörpers angeordnet ist, wobei sich der Hohlraum durchgehend in Längsrichtung des Fluidsammelkörpers erstreckt.

9. Medizinisches Produkt nach einem der vorhergehenden Ansprüche, **dadurch gekennzeichnet, dass** der Fluidkommunikationskörper derart in dem Fluidsammelkörper angeordnet ist, dass das distale Ende des Fluidsammelkörpers und das distale Ende des Fluidkommunikationskörpers fluchtend zueinander ausgebildet sind.

10. Medizinisches Produkt nach einem der vorhergehenden Ansprüche, **dadurch gekennzeichnet, dass** der Fluidsammelkörper als offenporöser Schwamm ausgebildet ist.

11. Medizinisches Produkt nach einem der vorhergehenden Ansprüche, **dadurch gekennzeichnet, dass** der Fluidsammelkörper aus Polyurethan, insbesondere linearem und vorzugsweise aliphatischem Polyurethan, gebildet ist.

12. Medizinisches Produkt nach einem der vorhergehenden Ansprüche, **dadurch gekennzeichnet, dass** der Fluidkommunikationskörper als Drainageschlauch ausgebildet ist.

## Revendications

1. Produit médical pour le drainage de l'accumulation pathologique de fluides tel que les accumulations d'ichor, comportant un élément (110) de collecte de fluide et un élément de communication de fluide,
dans lequel l'élément de communication de fluide (120) est au moins partiellement agencé à l'intérieur de l'élément de collecte de fluide et est relié à celui-ci de façon à conduire le fluide, un élément de tirage (130) destiné à placer le produit dans un corps humain ou animal étant agencé à l'intérieur de l'élément de communication de fluide, **caractérisé en ce que** l'élément de tirage fait saillie depuis une extrémité distale de l'élément de communication de fluide sous la forme d'une boucle.

2. Produit médical selon la revendication 1, **caractérisé en ce que** l'élément de communication de fluide s'étend sur toute la longueur de l'élément de collecte de fluide.

3. Produit médical selon la revendication 1 ou 2, **caractérisé en ce que** l'élément de tirage est agencé dans une cavité ou découpe de l'élément de communication de fluide, la cavité ou la découpe s'étendant continuellement le long de l'élément de communication de fluide.

4. Produit médical selon l'une quelconque des revendications précédentes, **caractérisé en ce que** l'élément de tirage est relié à l'élément de communication de fluide, de préférence à une extrémité proximale de celui-ci.

5. Produit médical selon l'une quelconque des revendications précédentes, **caractérisé en ce qu'**une extrémité fixe de la boucle fait saillie depuis une extrémité proximale de l'élément de communication de fluide.

6. Produit médical selon l'une quelconque des revendications précédentes, **caractérisé en ce que** l'élément de tirage est agencé comme textile, en particulier au moins un filament.

7. Produit médical selon l'une quelconque des revendications précédentes, **caractérisé en ce que** l'élément de tirage est agencé comme filament à boucle, ledit filament à boucle étant plus long que l'élément de communication de fluide.

8. Produit médical selon l'une quelconque des revendications précédentes, **caractérisé en ce que** l'élément de communication de fluide est agencé dans une cavité de l'élément de collecte de fluide, la cavité s'étendant de manière continue sur toute la longueur de l'élément de collecte de fluide.

9. Produit médical selon l'une quelconque des revendications précédentes, **caractérisé en ce que** l'élément de communication de fluide est agencé dans l'élément de collecte de fluide de telle manière que l'extrémité distale de l'élément de collecte de fluide et l'extrémité distale de l'élément de communication de fluide sont alignées l'une par rapport à l'autre (se terminent au même niveau).

10. Produit médical selon l'une quelconque des revendications précédentes, **caractérisé en ce que** l'élément de collecte de fluide est configuré sous forme d'éponge à pores ouverts.

11. Produit médical selon l'une quelconque des revendications précédentes, **caractérisé en ce que** l'élément de collecte de fluide est composé de polyuréthane, particulièrement linéaire et de préférence de polyuréthane aliphatique.

12. Produit médical selon l'une quelconque des revendications précédentes, **caractérisé en ce que** l'élément de communication de fluide est configuré sous la forme d'un tube de drainage.
